# EUROPÄISCHE PATENTSCHRIFT

(11) **EP 3 519 024 B1**
(45) Veröffentlichungstag und Bekanntmachung des Hinweises auf die Patenterteilung: **07.04.2021**
(21) Anmeldenummer: 17777261.3
(22) Anmeldetag: 29.09.2017
(51) Int. Cl.: A61M 11/00, A61M 15/00

(54) **ZERSTÄUBER, INSBESONDERE INHALATOR, FÜR DIE ZERSTÄUBUNG EINES FLÜSSIGEN WIRKSTOFFS ZU EINEM AEROSOL SOWIE EIN ENTSPRECHENDES VERFAHREN**
ATOMISER, IN PARTICULAR INHALER, FOR ATOMISING A LIQUID ACTIVE AGENT TO FORM AN AEROSOL AND A CORRESPONDING METHOD
PULVÉRISATEUR, EN PARTICULIER INHALATEUR, POUR PULVÉRISER UN PRINCIPE ACTIF LIQUIDE EN UN AÉROSOL ET PROCÉDÉ CORRESPONDANT

(30) Priorität: 30.09.2016 DE 102016118654
(43) Veröffentlichungstag der Anmeldung: 07.08.2019
(73) Patentinhaber: Softhale NV, 3590 Diepenbeek (BE)
(72) Erfinder: BARTELS, Frank, 45527 Hattingen (DE); RAWERT, Jürgen, 50933 Köln (DE)
(74) Vertreter: Pharma Patents International AG
(86) Internationale Anmeldenummer: PCT/EP2017/074775
(87) Internationale Veröffentlichungsnummer: WO 2018/060425

(56) Entgegenhaltungen:
- WO-A1-2014/019563
- US-A1- 2005 252 990
- US-A1- 2015 040 891

## Beschreibung

Die Erfindung geht aus von einem Zerstäuber, insbesondere einem Inhalator, für die Zerstäubung eines flüssigen Wirkstoffs zu einem Aerosol sowie von einem entsprechenden Verfahren. Der Zerstäuber weist ein Wirkstoffreservoir, eine Zerstäubungsdüse und eine Pumpenanordnung auf, wobei eine Saugseite der Pumpenanordnung in das Wirkstoffreservoir und eine Druckseite der Pumpenanordnung in die Zerstäubungsdüse mündet Die Pumpenanordnung weist einen in einem Zylinder in Axialrichtung des Zylinders verstellbaren Kolben auf. Ein derartiger Zerstäuber ist beispielsweise aus der US 7,104,470 B2 und aus der US 5,662,271 A bekannt Eine bespielhafte Zerstäubungsdüse ist aus der DE 10 2012 014 965 A1 bekannt. US2015/0040891 beschreibt eine Pumpenanordnung und einen Zylinder, der einen Kolben beinhaltet für die Zerstäubung eines flüssigen Wirkstoffes.

Bei den aus dem Stand der Technik bekannten Zerstäubern ist häufig ein Einlassventil vorgesehen, welches eine Pumpenkammer der Pumpenanordnung gegenüber dem Wirkstoffreservoir verschließt, wenn in der Pumpenkammer ein Überdruck für den Ausstoß eines Aerosols über die Zerstäubungsdüse erzeugt wird, so dass ein Zurückfließen des Wirkstoffes aus der Pumpenkammer in das Wirkstoffreservoir vermieden wird. Wenn die Pumpenkammer erneut befüllt werden soll, wird durch Verlagern des Kolbens ein Unterdruck in der Pumpenkammer erzeugt, wodurch der Wirkstoff aus dem Wirkstoffreservoir durch das offene Einlassventil in die Pumpenkammer transportiert wird. Während des Einsaugvorgangs ist ein der Zerstäubungsdüse beigeordnetes Auslassventil verschlossen, um ein Zurückfließen von Flüssigkeit oder Luft aus der Zerstäubungsdüse in die Pumpenkammer zu vermeiden.

Für das Ansaugen des Wirkstoffes aus dem Reservoir ist der Kolben der Pumpenanordnung häufig als eine Kapillare oder als Hohlkolben ausgeführt, mit einem Rückschlagventil, welches mit einer in der Kapillare beweglichen Kugel als Verschlusselement für die Kapillare dient, wenn in der Pumpenkammer ein Überdruck erzeugt wird, um über die Zerstäubungsdüse das Aerosol auszustoßen. Derartige als Kapillare ausgebildete Pumpenkolben sind jedoch aufwendig in der Herstellung und dementsprechend kostenintensiv.

Es ist daher die Aufgabe der Erfindung, einen Zerstäuber der eingangs beschriebenen Art sowie ein entsprechendes Verfahren derart weiterzuentwickeln, dass sie einfach und kostengünstig in der Herstellung sind.
Diese Aufgabe wird durch einen Zerstäuber mit den Merkmalen des Anspruchs 1 und ein entsprechendes Verfahren mit den Merkmalen des Anspruchs 12 gelöst. Die abhängigen Ansprüche betreffen jeweils vorteilhafte Ausführungsformen der Erfindung.

Demgemäß liegt der Kolben entlang seines Außenumfangs anteilig formschlüssig an einer Innenwand des Zylinders an, wobei weiterhin anteilig ein Spalt zwischen der Innenwand des Zylinders und dem Kolben ausgebildet ist. Der Kolben ist um seine Längsachse verdrehbar in dem Zylinder gelagert und zwischen einer Saugstellung, in der eine Pumpenkammer der Pumpenanordnung über den Spalt mit der Saugseite verbunden ist, und einer Pumpstellung, in der der Kolben die Saugseite verschließt, verstellbar.

Im Vergleich zu den aus dem Stand der Technik bekannten Zerstäubern ermöglicht die beschriebene Ausbildung der Pumpenanordnung insbesondere, dass der Kolben als ein massives Bauteil ausgebildet werden kann und nicht mehr als Kapillare mit integriertem (Kugel-) Rückschlagventil ausgebildet werden muss. Ein massiver und einteilig ausgebildeter Kolben ist in der Herstellung wesentlich einfacher und damit kostengünstiger realisierbar, als der zuvor beschriebene Kolben mit integrierter Kapillare sowie Kugelrückschlagventil.
Der Außenumfang des Kolbens kann, soweit der Kolben entlang des Außenumfangs formschlüssig an der Innenwand des Zylinders anliegt, einen Außendurchmesser aufweisen, der im Wesentlichen einem Innendurchmesser des Zylinders entspricht. Der Außendurchmesser des Kolbens kann dabei zwischen 0,1 mm und 3 mm, und besonders bevorzugt zwischen 0,2 und 1 mm betragen. Entlang der formschlüssig aneinandergrenzenden Flächen von Kolben und Zylinder kann der Kolben in dem Zylinder zwischen der Pumpstellung und der Saugstellung geführt sein.

Der Außenumfang des Kolbens kann, soweit der Spalt zwischen der Innenwand des Zylinders und dem Außenumfang des Kolbens ausgebildet ist, unrund sein und insbesondere eine Abflachung aufweisen. Der Kolben kann dabei beispielsweise als ein Rundstab ausgebildet sein, der an einem Ende, mit dem er in die Pumpenkammer hineinragt, einseitig abgeflacht ist, wobei die Abflachung beispielsweise durch lokale, seitliche Materialwegnahme, beispielsweise Anfräsen des Rundstabes, ausgebildet sein kann.

Die Saugseite der Pumpenanordnung kann über einen sich in Radialrichtung des Zylinders erstreckenden und in der Saugstellung in den Spalt mündenden Durchlass mit der Pumpenkammer verbunden sein.

Dabei kann eine innere Mantelfläche am Innenumfang des Zylinders über ihre gesamte Fläche einen konstanten Krümmungsradius aufweisen und nur über den Durchlass unterbrochen sein. An einem Übergang zwischen dem Durchlass und der Innenwand des Zylinders kann ein den Durchlass umschließendes Dichtelement angeordnet sein, um die Abdichtung des Durchlasses gegenüber der Pumpenkammer in der Pumpstellung des Kolbens weiter zu verbessern.

In der Saugstellung kann der Spalt dem Durchlass zugewandt und in der Pumpstellung von dem Durchlass abgewandt sein, wobei die Saugstellung und die Pumpstellung zwei um im Wesentlichen um 180° zueinander verdrehte Stellpositionen des Kolbens sind. Für das Verschließen des Durchlasses in der Pumpstellung ist es nicht zwingend erforderlich, dass der Kolben gegenüber der Saugstellung, in der der Spalt dem Durchlass zugewandt ist, um 180° verdreht wird. Je nach realisierbarer Fertigungstoleranz zwischen Außenumfang des Kolbens und Innenumfang des Zylinders und dadurch erreichbarer Druckdichtigkeit zwischen Durchlass und Pumpenkammer in der Pumpstellung kann die Pumpstellung gegenüber der Saugstellung auch um weniger als 180°, beispielsweise um lediglich 90° verdreht sein.

Der Kolben kann beispielsweise ein Kreiszylinder sein, der an einer parallel zur Längsachse des Kolbens verlaufenden Seite eine Ausnehmung aufweist. Insbesondere kann der Kolben ein einseitig abgeflachter Kreiszylinder sein. Eine Verbindungsfläche zwischen der kreisförmigen Mantelfläche des Kolbens und der Ausnehmung oder einer abgeflachten Seite kann abgerundet sein. Diese Ausführungsform trägt dazu bei, dass ein eventuell vorgesehenes Dichtelement, welches den Durchlass an der Innenwand des Zylinders umschließt, während des Verdrehens des Kolbens zwischen der Saugstellung und der Pumpstellung nicht beschädigt wird.
Gemäß einem anderen Aspekt betrifft die Erfindung ein Verfahren für den Betrieb eines Zerstäubers der zuvor beschriebenen Art. Das Verfahren weist das Verlagern des Kolbens in Bezug auf den Zylinder in Längsrichtung von Kolben und Zylinder zwischen einer ausgezogenen Position und einer eingeschobenen Position auf.

Dabei kann vorgesehen sein, dass bei dem Verlagern des Kolbens aus der ausgezogenen Position in die eingeschobene Position der Kolben die Saugseite verschließt, während bei dem Verlagern des Kolbens aus der eingeschobenen Position in die ausgezogene Position der Spalt die Saugseite mit der Pumpenkammer verbindet.

Für das Verschließen der Saugseite kann der Kolben um seine Längsachse von der Saugstellung in die Pumpstellung verdreht werden.

Das Verfahren kann weiterhin die Schritte aufweisen:
a) Verlagern des Kolbens entlang seiner Längsrichtung aus der eingeschobenen Position in die ausgezogene Position, wobei sich der Kolben in der Saugposition befindet und wobei ein Unterdruck in der Pumpenkammer erzeugt wird, sodass ein flüssiger Wirkstoff aus dem Wirkstoffreservoir in die Pumpenkammer gezogen wird, danach
b) Drehen des Kolbens aus der Saugposition in die Pumpposition; danach
c) Verlagern des Kolbens von der ausgezogenen Position in die eingeschobene Position, wobei sich der Kolben weiterhin in der Pumpposition befindet, und wobei ein Überdruck in der Pumpenkammer erzeugt und der flüssige Wirkstoff in der Pumpenkammer über die Zerstäubungsdüse aus dem Zerstäuber ausgestoßen wird; danach
d) Drehen des Kolbens aus der Pumpposition in die Saugposition.
Um weiteres Aerosol auszustoßen können die Schritte a) bis d) entsprechend der gewünschten Aerosolmenge mindestens einmal wiederholt werden.

### Beschreibung der Figuren

Weitere Einzelheiten der Erfindung werden anhand der nachstehenden Figuren erläutert. Dabei zeigt:
- Figur 1: einen Zerstäuber gemäß dem Stand der Technik;
- Figur 2: eine schematische Querschnittsansicht senkrecht zu Längsachse einer Pumpenanordnung einer Ausführungsform eines erfindungsgemäßen Zerstäubers;
- Figuren 3 bis 6: schematisch eine Ausführungsform eines erfindungsgemäßen Zerstäubers in verschiedenen Stellpositionen des Kolbens in Bezug auf den Zylinder.

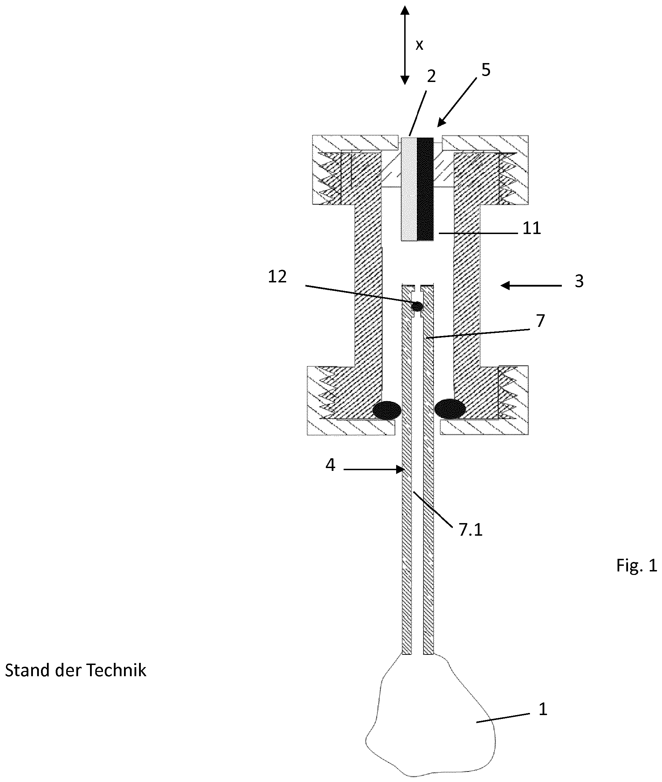

Die Figur 1 zeigt einen Zerstäuber nach dem Stand der Technik. Dieser besteht im Wesentlichen aus einem Wirkstoffreservoir 1 und einer Zerstäubungsdüse 2, die über eine Pumpenanordnung 3 fluidisch miteinander verbunden sind. Über die Pumpenanordnung 3 kann ein in dem Wirkstoffreservoir 1 vorgehaltener Wirkstoff unter Druck durch die Zerstäubungsdüse 2 hindurchgepresst werden, so dass dieses in feinste Partikel zerstäubt wird und ein Aerosol bildet. Eine geeignete Zerstäubungsdüse ist in der DE 10 2012 014 965 A1 beschrieben. Der Zerstäuber kann beispielsweise als Inhalator verwendet werden.

Die Pumpenanordnung 3 weist eine Saugseite 4 und eine Druckseite 5 auf, wobei die Saugseite über eine Kapillare 7.1 in einem Kolben 7 der Pumpenanordnung 3 mit dem Wirkstoffreservoir 1 fluidisch verbunden ist. Des Weiteren ist die Pumpenkammer 11 mit der Zerstäubungsdüse 2 fluidisch verbunden.

Der Kolben 7 ist in dem Zylinder 6 in seiner Längsrichtung x verschieblich. An seinem in die Pumpenkammer 11 hineinragenden Ende weist der Kolben 7 ein Kugelventil 12 auf, welches die Kapillare 7.1 freigibt, wenn der Kolben 7 in einer Saugbewegung aus der Pumpenkammer 11 zumindest teilweise herausgezogen wird, und dass die Kapillare 7.1 verschließt, wenn der Kolben 7 in einer Pumpbewegung weiter in die Pumpenkammer 1 eingeschoben wird. Wenn somit der Kolben 7 aus der Pumpenkammer 11 zumindest teilweise herausgezogen wird, kann durch den in der Pumpenkammer 11 entstehenden Unterdruck ein Wirkstoff aus dem Wirkstoffreservoir 1 durch die Kapillare 7.1 in die Pumpenkammer 11 eintreten.

Wenn daraufhin in einem folgenden Schritt der Kolben 7 wieder weiter in die Pumpenkammer 11 eingeschoben wird, verschließt das Kugelventil die Kapillare 7.1 aufgrund des dabei in der Pumpenkammer 11 entstehenden Überdrucks, so dass der in der Pumpenkammer 11 befindliche Wirkstoff nur über die Zerstäubungsdüse 2 aus der Pumpenkammer 11 heraustreten und insbesondere nicht durch die Kapillare 7.1 zurück in das Wirkstoffreservoir 1 zurückfließen kann.

Im Bereich der Wirkstoffzerstäuber weisen die Kolben 7 üblicherweise einen Durchmesser von ungefähr 0,9 bis 1,5 mm auf, so dass die sich im Innern des Kolbens 7 in Längsrichtung des Kolbens erstreckende Kapillare einen entsprechend nochmals geringeren Durchmesser aufweisen muss. Hoch komplex ist dabei insbesondere die Ausbildung des Kugelrückschlagventils 12, wobei zur Gewährleistung der Funktionstüchtigkeit des Ventils 12 Fertigungstoleranzen im Mikrometerbereich eingehalten werden müssen, wodurch der Zerstäuber sehr aufwendig in der Herstellung und damit kostenintensiv ist.

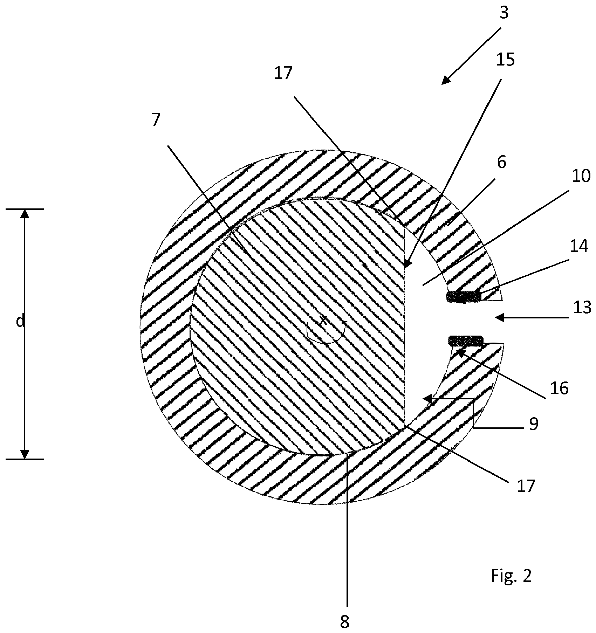

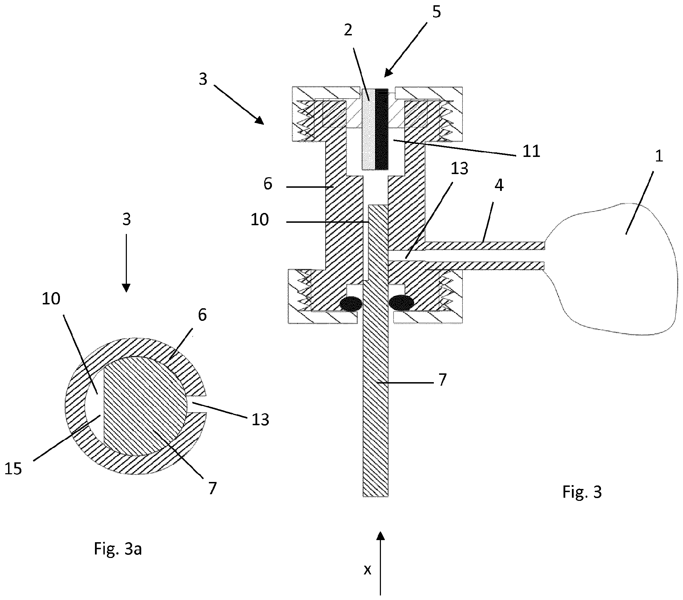

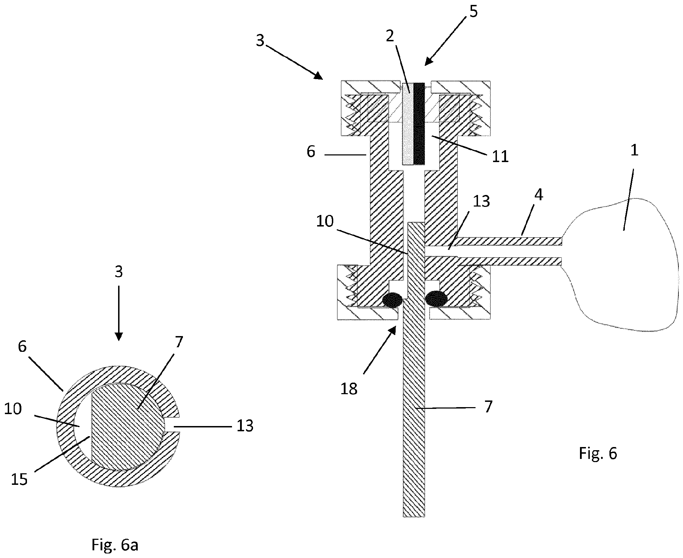

In Figur 2 ist ein Querschnitt senkrecht zur Längsrichtung des Kolbens 7 und des Zylinders 6 einer Pumpenanordnung 3 gemäß einer Ausführungsform der Erfindung gezeigt. Demgemäß weist der Kolben entlang seines Außenumfangs 8 eine Ausnehmung 10 auf, so dass der Kolben entlang seines Außenumfangs anteilig formschlüssig an der Innenwand 9 des Zylinders 6 anliegt, und anteilig einen Spalt 10 zwischen der Innenwand 9 des Zylinders und dem Kolben 7 ausbildet. Der Kolben 7 ist um seine Längsachse x, die sich in der Darstellung gemäß Figur 2 senkrecht zur Zeichnungsebene erstreckt, verdrehbar in dem Zylinder 6 gelagert. In Figur 2 ist der Kolben 7 in Bezug auf den Zylinder 6 in der Saugstellung angeordnet. In der Saugstellung ist der Spalt 10 einer Seite des Zylinders 6 zugewandt, durch welche sich in Radialrichtung des Zylinders 6 ein Durchlass 13 hindurch erstreckt. Über den Durchlass 13 kann die Saugseite 4 der Pumpenanordnung 3 angeschlossen sein, so dass über den Durchlass 13 und den Spalt 10 eine fluidische Verbindung zwischen einem über die Saugseite 4 angeschlossenen Wirkstoffreservoir 1 und einer Pumpenkammer 11 der Pumpenanordnung 3 hergestellt werden kann (in Figur 2 nicht dargestellt, siehe Figuren 3 bis 6).

Der Kolben 7 ist um seine senkrecht zur Zeichnungsebene von Figur 2 verlaufende Längsachse x verdrehbar. Wenn der Kolben 7 beispielsweise gegenüber der in Figur 2 gezeigten Stellung um 180° um die Achse x verdreht wird, so ist der Spalt 10 einem von dem Durchlass 13 abgewandten Seitenwandabschnitt des Zylinders 6 zugewandt. In diesem Fall verschließt der Zylinder 7 mit seinem an die Ausnehmung 15 angrenzenden Außenumfang den Durchlass 13. Um die Abdichtung zwischen dem Innenumfang 9 des Zylinders 6 und dem Außenumfang 8 des Kolbens 7 im Bereich des Durchlasses 13 zu verbessern, weist der Durchlass 13 an seinem Übergang zur Innenwand 9 des Zylinders 6 ein Dichtelement 14 auf. Damit durch das Verdrehen des Kolbens 7 innerhalb des Zylinders 6 das Dichtelement 14 nicht beschädigt wird, weisen die Verbindungsflächen 17, welche den kreissymmetrischen Außenumfang 8 des Kolbens mit der Ausnehmung 5 verbinden, jeweils eine Abrundung auf.

Die den Spalt 10 zwischen dem Kolben 7 und der Innenwand 9 des Zylinders 6 ausbildende Ausnehmung 15 ist als eine seitliche Abflachung des ansonsten im Wesentlichen kreiszylinderförmigen Kolbens 7 ausgebildet.

Die Figuren 3 bis 6 zeigen vier verschiedene Stellpositionen des Kolbens 7 in Bezug auf den Zylinder 6. In den Figuren 3 und 3a ist der Spalt 10 abgewandt von dem Durchlass 13 angeordnet, so dass der Kolben 7 den Durchlass 13 verschließt. Des Weiteren wird der Kolben 7 entlang seiner Längsrichtung x aus einer ausgezogenen Position in eine eingeschobene Position verlagert. Da der Kolben 7 den Durchlass 13 verschließt, kann ein Überdruck in der Pumpenkammer 11 erzeugt werden, so dass ein sich in der Pumpenkammer befindender flüssiger Wirkstoff über die Zerstäubungsdüse 2 aus dem Zerstäuber ausgestoßen wird.

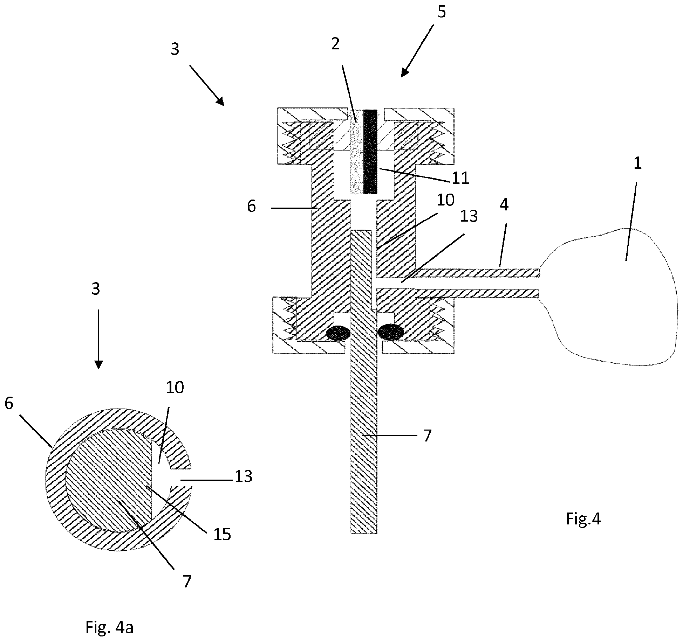

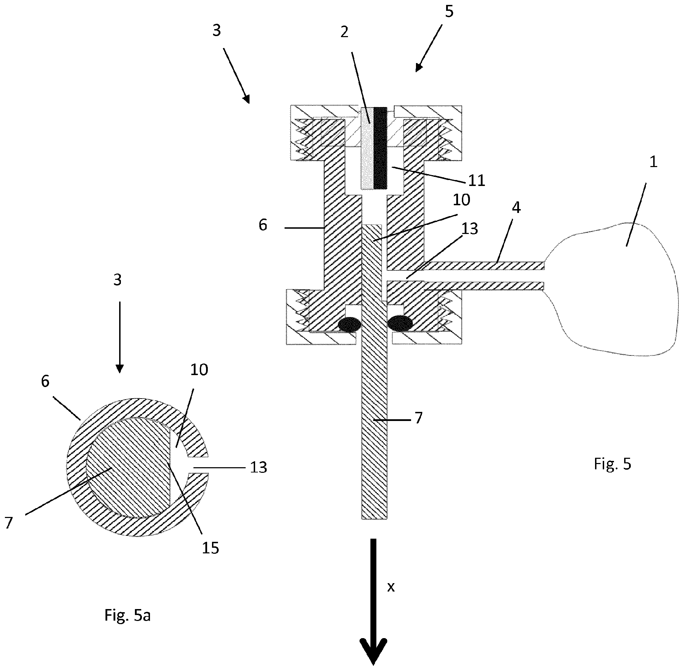

Wenn sich der Kolben 7 in der eingeschobenen Position befindet, wird der Kolben, wie in den Figuren 4 und 4a dargestellt ist, aus der in den Figuren 3 und 3a gezeigten Pumpposition in die in den Figuren 4 und 4a gezeigte Saugposition gedreht, bei der der Spalt 10 dem Durchlass 13 zugewandt und somit eine fluidische Verbindung zwischen der Pumpenkammer 11 und dem Wirkstoffreservoir 1 über den Spalt 10, den Durchlass 13 und die Saugseite 4 herstellt ist. In dieser rotativen Ausrichtung des Kolbens 7 in Bezug auf den Zylinder 6 kann für das Befüllen der Pumpenkammer 11 der Kolben 7 zumindest teilweise aus der Pumpenkammer 11 herausgezogen werden, so dass in der Pumpenkammer 11 ein Unterdruck entsteht. Dies ist in den Figuren 5 und 5a gezeigt. Aufgrund des Unterdrucks kann ein sich in dem Wirkstoffreservoir 1 befindender Wirkstoff über die Saugseite 4, den Durchlass 13 und den Spalt 10 in die Pumpenkammer 11 befördert werden. Die Zerstäubungsdüse 2 kann dazu ein Rückschlagventil aufweisen, welches bei einem Unterdruck in der Pumpenkammer 11 die Düse 2 gegenüber der Außenumgebung der Pumpenanordnung 3 verschließt.

Nachdem die Pumpenkammer 11 zumindest teilweise mit Wirkstoff befüllt worden ist, wird der Kolben 7 in dem Zylinder 6 wieder um 180° verdreht, so dass der Kolben 7 den Durchlass 13 in den Zylinder verschließt.

Die Pumpenanordnung 3 ist somit dazu vorbereitet, um entsprechend der in den Figuren 3 und 3a gezeigten Situation durch erneutes Verlagern des Kolbens 7 entlang der Längsrichtung x aus der ausgezogenen Position in die eingeschobene Position einen Überdruck in der Pumpenkammer 11 zu erzeugen, so dass weiterer flüssiger Wirkstoff über die Zerstäubungsdüse 2 aus dem Zerstäuber ausgestoßen wird.

Der Kolben 7 ist bei den in den Figuren 2 bis 6a gezeigten Ausführungsformen als ein im Querschnitt senkrecht zur Längsrichtung des Kolbens einseitig abgeflachter Kreiszylinder ausgebildet und kann somit durch einseitiges, abschnittsweises Abflachen eines kreiszylinderförmigen Kolbens einfach und damit kostengünstig hergestellt werden. Es ist weiterhin zu erkennen, dass die Ausnehmung 15 nur an dem in die Pumpenkammer 11 hineinragenden Ende des Kolbens 7 ausgebildet ist, wobei der Kolben insbesondere in einem Dichtungsbereich 18, in dem der Kolben 7 gegenüber der Pumpenkammer 11 abgedichtet ist, im Querschnitt senkrecht zu seiner Längsachse kreissymmetrisch ist, so dass der Kolben 7 gegenüber der Pumpenkammer 11 mit einfachen Dichtmitteln, etwa mit einem O-Ring, abgedichtet werden kann.

Die in der vorstehenden Beschreibung, in der Zeichnung sowie in den Ansprüchen offenbarten Merkmale der Erfindung können sowohl einzeln als auch in beliebiger Kombination für die Verwirklichung der Erfindung wesentlich sein.

### Bezugszeichenliste

- 1: Wirkstoffreservoir
- 2: Zerstäubungsdüse
- 3: Pumpenanordnung
- 4: Saugseite der Pumpenanordnung
- 5: Druckseite der Pumpenanordnung
- 6: Zylinder
- 7: Kolben
- 7.1: Kapillare
- 8: Außenumfang
- 9: Innenwand
- 10: Spalt
- 11: Pumpenkammer
- 12: Kugelventil
- 13: Durchlass
- 14: Dichtelement
- 15: Ausnehmung
- 16: Übergang
- 17: Verbindungsfläche
- 18: Dichtungsbereich
- d: Außendurchmesser des Kolbens
- x: Längsachse

## Patentansprüche

1. Zerstäuber, insbesondere Inhalator, für die Zerstäubung eines flüssigen Wirkstoffs zu einem Aerosol, mit einem Wirkstoffreservoir (1), einer Zerstäubungsdüse (2) und einer Pumpenanordnung (3), wobei eine Saugseite (4) der Pumpenanordnung (3) in das Wirkstoffreservoir (1) und eine Druckseite (5) der Pumpenanordnung (3) in die Zerstäubungsdüse (2) mündet, und wobei die Pumpenanordnung (3) einen in einem Zylinder (6) in Axialrichtung (x) des Zylinders (6) verstellbaren Kolben (7) aufweist, **dadurch gekennzeichnet, dass** der Kolben (7) entlang seines Außenumfangs (8) anteilig formschlüssig an einer Innenwand (9) des Zylinders (6) anliegt und anteilig ein Spalt (10) zwischen der Innenwand (9) des Zylinders (6) und dem Kolben (7) ausgebildet ist, wobei der Kolben (7) um seine Längsachse (x) verdrehbar in dem Zylinder (6) gelagert und zwischen einer Saugstellung, in der eine Pumpenkammer (11) der Pumpenanordnung (3) über den Spalt (10) mit der Saugseite (4) verbunden ist, und einer Pumpstellung, in der der Kolben (7) die Saugseite (4) verschließt, verstellbar ist

2. Zerstäuber nach Anspruch 1, bei dem der Kolben (7) massiv ist

3. Zerstäuber nach Anspruch 1 oder 2, bei dem der Außenumfang (8) des Kolbens (7), soweit der Kolben (7) entlang des Außenumfangs (8) formschlüssig an der Innenwand (9) des Zylinders (6) anliegt, einen Außendurchmesser (d) aufweist, der im Wesentlichen einem Innendurchmesser des Zylinders (6) entspricht, wobei der Außendurchmesser (d) des Kolbens vorzugsweise zwischen 0,1 mm und 3 mm, und besonders bevorzugt zwischen 0,2 und 1 mm beträgt

4. Zerstäuber nach einem der vorangegangenen Ansprüche, bei dem der Außenumfang (8) des Kolbens (7), soweit der Spalt (10) zwischen der Innenwand (9) des Zylinders (6) und dem Außenumfang (8) des Kolbens (7) ausgebildet ist, unrund ist und insbesondere abgeflacht ist.

5. Zerstäuber nach einem der vorangegangenen Ansprüche, bei dem die Saugseite (4) der Pumpenanordnung (3) über einen sich in Radialrichtung des Zylinders (6) erstreckenden und in der Saugstellung in den Spalt (10) mündenden Durchlass (13) mit der Pumpenkammer (11) verbunden ist.

6. Zerstäuber nach Anspruch 5, bei dem eine innere Mantelfläche (13) am Innenumfang des Zylinders (6) über ihre gesamte Fläche einen konstanten Krümmungsradius aufweist und nur über den Durchlass (13) unterbrochen ist

7. Zerstäuber nach Anspruch 5 oder 6, bei dem an einem Übergang (16) zwischen dem Durchlass (13) und der Innenwand (9) des Zylinders (6) ein den Durchlass (13) umschließendes Dichtelement (14) angeordnet ist.

8. Zerstäuber nach einem der Ansprüche 5 bis 7, bei dem in der Saugstellung der Spalt (10) dem Durchlass (13) zugewandt und in der Pumpstellung von dem Durchlass (13) abgewandt ist, wobei die Saugstellung und die Pumpstellung zwei um im Wesentlichen um 180° zueinander verdrehte Stellpositionen des Kolbens (7) sind.

9. Zerstäuber nach einem der vorangegangenen Ansprüche, bei dem der Kolben (7) ein Kreiszylinder ist, der an einer parallel zur Längsachse (x) des Kolbens (7) verlaufenden Seite eine Ausnehmung (15) aufweist.

10. Zerstäuber nach Anspruch 9, bei dem der Kolben (7) ein einseitig abgeflachter Kreiszylinder ist

11. Zerstäuber nach Anspruch 9 oder 10, bei dem eine Verbindungsfläche (17) zwischen der kreisförmigen Mantelfläche (13) des Kolbens (7) und der Ausnehmung (15) oder einer abgeflachten Seite abgerundet ist.

12. Verfahren für den Betrieb eines Zerstäubers nach einem der vorangegangenen Ansprüche, **dadurch gekennzeichnet dass** es das Verlagern des Kolbens (7) in Bezug auf den Zylinder (6) in Längsrichtung (x) von Kolben (7) und Zylinder (6) zwischen einer ausgezogenen Position in eine eingeschobene Position aufweist

13. Verfahren nach Anspruch 12, bei dem bei dem Verlagern des Kolbens (7) aus der ausgezogenen Position in die eingeschobene Position der Kolben (7) die Saugseite (4) verschließt, und bei dem bei dem Verlagern des Kolbens (7) aus der eingeschobenen Position in die ausgezogene Position der Spalt (10) die Saugseite (4) mit der Pumpenkammer (11) verbindet

## Claims

1. Atomizer, in particular an inhaler, for atomizing a liquid active substance to form an aerosol, having an active substance reservoir (1), an atomizing nozzle (2) and a pump arrangement (3), wherein a suction side (4) of the pump arrangement (3) opens into the active substance reservoir (1) and a pressure side (5) of the pump arrangement (3) opens into the atomizing nozzle (2), and wherein the pump arrangement (3) has a piston (7) which is adjustable in a cylinder (6) in an axial direction (x) of the cylinder (6), **characterized in that** the piston (7), along its outer circumference (8), bears in part with form-fit engagement on an inner wall (9) of the cylinder (6), and a gap (10) is formed in part between the inner wall (9) of the cylinder (6) and the piston (7), wherein the piston (7) is mounted rotatably about its longitudinal axis (x) in the cylinder (6) and is adjustable between a suction position, in which a pump chamber (11) of the pump arrangement (3) is connected to the suction side (4) via the gap (10), and a pump position, in which the piston (7) closes the suction side (4).

2. Atomizer according to Claim 1, in which the piston (7) is solid.

3. Atomizer according to Claim 1 or 2, in which, where the piston (7) bears along the outer circumference (8) with form-fit engagement on the inner wall (9) of the cylinder (6), the outer circumference (8) of the piston (7) has an external diameter (d) which corresponds substantially to an internal diameter of the cylinder (6), wherein the external diameter (d) of the piston is preferably between 0.1 mm and 3 mm, and particularly preferably between 0.2 and 1 mm.

4. Atomizer according to one of the preceding claims, in which, where the gap (10) is formed between the inner wall (9) of the cylinder (6) and the outer circumference (8) of the piston (7), the outer circumference (8) of the piston (7) is non-round and is in particular flattened.

5. Atomizer according to one of the preceding claims, in which the suction side (4) of the pump arrangement (3) is connected to the pump chamber (11) via a passage (13) extending in the radial direction of the cylinder (6) and opening into the gap (10) in the suction position.

6. Atomizer according to Claim 5, in which an inner shell surface (13) on the inner circumference of the cylinder (6) has a constant radius of curvature across its entire surface area and is interrupted only by the passage (13).

7. Atomizer according to Claim 5 or 6, in which a sealing element (14) surrounding the passage (13) is arranged at a transition (16) between the passage (13) and the inner wall (9) of the cylinder (6).

8. Atomizer according to one of Claims 5 to 7, in which the gap (10), in the suction position, faces towards the passage (13) and, in the pump position, faces away from the passage (13), wherein the suction position and the pump position are two adjustment positions of the piston (7) that are rotated in relation to each other by substantially 180°.

9. Atomizer according to one of the preceding claims, in which the piston (7) is a circular cylinder which has a recess (15) on a side running parallel to the longitudinal axis (x) of the piston (7).

10. Atomizer according to Claim 9, in which the piston (7) is a circular cylinder flattened at one side.

11. Atomizer according to Claim 9 or 10, in which a connection surface (17) between the circular shell surface (13) of the piston (7) and the recess (15) or a flattened side is rounded.

12. Method for operating an atomizer according to one of the preceding claims, **characterized in that** it comprises moving the piston (7) in relation to the cylinder (6), in the longitudinal direction (x) of piston (7) and cylinder (6), between an extended position and an inserted position.

13. Method according to Claim 12, in which, when the piston (7) is moved from the extended position to the inserted position, the piston (7) closes the suction side (4), and in which, when the piston (7) is moved from the inserted position to the extended position, the gap (10) connects the suction side (4) to the pump chamber (11).

## Revendications

1. Nébulisateur, en particulier inhalateur, destiné à nébuliser un principe actif liquide pour former un aérosol, le nébulisateur comprenant un réservoir de principe actif (1), une buse de nébulisation (2) et un ensemble de pompage (3), un côté aspiration (4) de l'ensemble de pompage (3) débouchant dans le réservoir de principe actif (1) et un côté sous pression (5) de l'ensemble de pompage (3) débouchant dans la buse de nébulisation (2), et l'ensemble de pompage (3) comportant un piston (7) déplaçable dans un cylindre (6) dans la direction axiale (x) du cylindre (6), **caractérisé en ce que** le piston (7) venant en appui proportionnellement par complémentarité de formes sur une paroi intérieure (9) du cylindre (6) le long de sa circonférence extérieure (8) et un espace (10) est ménagé proportionnellement entre la paroi intérieure (9) du cylindre (6) et le piston (7), le piston (7) étant monté dans le cylindre (6) de manière à pouvoir tourner sur son axe longitudinal (x) et étant déplaçable une position d'aspiration, dans laquelle une chambre de pompage (11) de l'ensemble de pompage (3) est reliée au côté aspiration (4) par le biais de l'espace (10), et une position de pompage dans laquelle le piston (7) ferme le côté aspiration (4).

2. Nébulisateur selon la revendication 1, dans lequel le piston (7) est plein.

3. Nébulisateur selon la revendication 1 ou 2, dans lequel la circonférence extérieure (8) du piston (7), dans la mesure où le piston (7) vient en appui par complémentarité de formes sur la paroi intérieure (9) du cylindre (6) le long de la circonférence extérieure (8), a un diamètre extérieur (d) qui correspond sensiblement à un diamètre intérieur du cylindre (6), le diamètre extérieur (d) du piston étant de préférence compris entre 0,1 mm et 3 mm, et de manière particulièrement préférée entre 0,2 et 1 mm.

4. Nébulisateur selon l'une des revendications précédentes, dans lequel la circonférence extérieure (8) du piston (7), dans la mesure où l'espace (10) est ménagé entre la paroi intérieure (9) du cylindre (6) et la circonférence extérieure (8) du piston (7), n'est pas ronde et est en particulier aplatie.

5. Nébulisateur selon l'une des revendications précédentes, dans lequel le côté aspiration (4) de l'ensemble de pompage (3) est relié à la chambre de pompe (11) par le biais d'un passage (13) qui s'étend dans la direction radiale du cylindre (6) et qui débouche, dans la position d'aspiration, dans l'espace (10).

6. Nébulisateur selon la revendication 5, dans lequel une surface latérale intérieure (13) au niveau de la circonférence intérieure du cylindre (6) a un rayon de courbure constant sur toute sa surface et n'est interrompue que par le passage (13).

7. Nébulisateur selon la revendication 5 ou 6, dans lequel un élément d'étanchéité (14), entourant le passage (13), est disposé au niveau d'une transition (16) entre le passage (13) et la paroi intérieure (9) du cylindre (6).

8. Nébulisateur selon l'une des revendications 5 à 7, dans lequel l'espace (10) est dirigé vers le passage (13) dans la position d'aspiration et à l'opposé du passage (13) dans la position de pompage, la position d'aspiration et la position de pompage étant deux positions de réglage du piston (7) tournées sensiblement à 180° l'une par rapport à l'autre.

9. Nébulisateur selon l'une des revendications précédentes, dans lequel le piston (7) est un cylindre circulaire qui comporte un évidement (15) sur un côté qui s'étend parallèlement à l'axe longitudinal (x) du piston (7).

10. Nébulisateur selon la revendication 9, dans lequel le piston (7) est un cylindre circulaire aplati d'un côté.

11. Nébulisateur selon la revendication 9 ou 10, dans lequel une surface de liaison (17) située entre la surface latérale circulaire (13) du piston (7) et l'évidement (15) ou un côté aplati est arrondie.

12. Procédé de fonctionnement d'un nébulisateur selon l'une des revendications précédentes, **caractérisé en ce qu'**il comporte le déplacement du piston (7) par rapport au cylindre (6) dans la direction longitudinale (x) du piston (7) et du cylindre (6) entre une position sortie et une position rentrée.

13. Procédé selon la revendication 12, dans lequel, lorsque le piston (7) est déplacé de la position sortie à la position rentrée, le piston (7) ferme le côté aspiration (4) et dans lequel, lorsque le piston (7) est déplacé de la position rentrée à la position sortie, l'espace (10) relie le côté aspiration (4) à la chambre de pompage (11).
